(19) Europäisches Patentamt
European Patent Office
Office européen des brevets

(11) EP 3 445 415 B1

(12) **EUROPÄISCHE PATENTSCHRIFT**

(45) Veröffentlichungstag und Bekanntmachung des Hinweises auf die Patenterteilung:
**16.12.2020 Patentblatt 2020/51**

(21) Anmeldenummer: **17719993.2**

(22) Anmeldetag: **23.03.2017**

(51) Int Cl.:
***A61L 27/50*** *(2006.01)*

(86) Internationale Anmeldenummer:
**PCT/DE2017/100238**

(87) Internationale Veröffentlichungsnummer:
**WO 2017/182024 (26.10.2017 Gazette 2017/43)**

(54) **SCHICHT MIT VARIABLER FESTIGKEIT**

LAYER HAVING VARIABLE STRENGTH

COUCHE À RÉSISTANCE VARIABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2016 DE 102016107480**

(43) Veröffentlichungstag der Anmeldung:
**27.02.2019 Patentblatt 2019/09**

(73) Patentinhaber: **Christian-Albrechts-Universität zu Kiel**
**24118 Kiel (DE)**

(72) Erfinder:
- **TIMMERMANN, Michael**
**24105 Kiel (DE)**
- **GUTEKUNST, Sören**
**67117 Limburgerhof (DE)**
- **SELHUBER-UNKEL, Christine**
**24107 Kiel (DE)**
- **QUANDT, Eckhard**
**24226 Heikendorf (DE)**

(74) Vertreter: **Hansen, Jochen**
**Hansen und Heeschen**
**Patentanwälte**
**Eisenbahnstrasse 5**
**21680 Stade (DE)**

(56) Entgegenhaltungen:
**US-A1- 2003 055 496**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 445 415 B1

## Beschreibung

Die Erfindung betrifft eine Schicht, die in der Lage ist, bei Einwirken einer äußeren Kraft, ihre Festigkeit sprungartig zu erhöhen, sobald die äußere Kraft einen bestimmten Wert erreicht hat, und bei Nachlassen dieser äußeren Kraft wieder in den ursprünglichen Zustand zurückzukehren, des Weiteren betrifft die Erfindung ein Verfahren zur Herstellung dieser Schicht sowie die Verwendung der Schicht als schockabsorbierendes Mittel, zur Erzeugung von Gefäßprothesen mit nichtlinear elastischem Verhalten und für flexible Anzeigevorrichtungen. Das Verhalten bei Einwirken einer äußeren Kraft, die Festigkeit zu erhöhen ist als "Strain-Stiffening" bekannt. Einige Körpergewebe, wie zu Beispiel die Aorta zeigen "Strain-Stiffening"-Verhalten. Entsprechende Materialien können besseren Ersatz bieten, als Materialien mit linearem Elastizitätsverhalten (oder sogar "Strain-Softening"). [Qi Wen, Paul A. Janmey, Effects of non-linearity on cell-ECM interactions, Experimental Cell Research, Volume 319, Issue 16, 1 October 2013, Pages 2481-2489, ISSN 0014-4827, http://dx.doi.org/10.1016/j.yexcr.2013.05.017].

Aus den Druckschriften "Self-Healing Polymers: From Principles to Applications, First Edition. Edited by Wolfgang H. Binder © 2013 Wiley-VCH Verlag GmbH & Co. KGaA. Published 2013 by Wiley-VCH Verlag GmbH & Co. und Kim et al. Biomicrofluidics 7, 041501 (2013) doi: 10.1063/1.4816934 und Pence et al. Biomater. Sci., 2014, 2, 1296-1304 (DOI: 10.1039/c4bm00193a) können die Substanzen für die erfindungsgemäße Schicht durch den zuständigen Fachmann leicht ausgewählt werden, wobei der Fachmann über die Befähigung zur Auswahl der richtigen Substanzen verfügt.

Die US 2003/0055496 A1 schlägt eine Gefäßprothese mit nicht linear elastischem Verhalten vor, bei der das nicht linear elastische Verhalten durch textile Verhakungen der äußeren Schicht verursacht wird. Dem aus medizinischer Sicht sehr erstrebenswerten nicht linear elastischen Verhalten, sind bei dem System aber Grenzen durch die Geometrie und Größe der textilen Verhakungen gesetzt.

Die US 2010/0205722 beschreibt eine schockabsorbierende Schicht bei der mehrere vereinzelte, regelmäßig angeordnete, flexible Elemente, durch Berührung mit einem weniger flexiblen Gitter kommt es zu einer Verteilung der Anprallkraft über eine größere Fläche und somit zu einer stoß- und schockabsorbierenden Wirkung. Nachteil dieser Systeme ist die geringe Flexibilität der Systeme.

Die DE 60222805 T2 schlägt daher den Einsatz von dilatanten Fluiden im Rahmen eines energieabsorbierenden Verbundstoffes vor. Das System ist durch den Einsatz des Fluides limitiert und nicht für sehr dünne Schichten geeignet.

Aus der US 8,072,437 B2 sind flexible Multitouch Displays bekannt, hier wäre zum Schutz der Sensoren eine Schicht, die bei Einwirken einer äußeren Kraft sich ab einem bestimmten Wert verfestigt und bei Nachlassen dieser äußeren Kraft sofort wieder die ursprüngliche Flexibilität erreicht von großen Vorteil. Bei diesen Systemen ist es entscheidend wichtig, die Dimensionen klein, vorzugsweise in einem Bereich von kleiner 100$\mu$m halten zu können.

Daher ist es Aufgabe der Erfindung eine Schicht, die in der Lage ist, bei Einwirken einer äußeren Kraft, ihre Festigkeit sprungartig zu erhöhen, sobald die äußere Kraft einen bestimmten Wert erreicht hat, und bei Nachlassen dieser äußeren Kraft wieder in den ursprünglichen Zustand zurückzukehren bereitzustellen.

**Des Weiteren ist es Aufgabe** der Erfindung eine Schicht, die in der Lage ist, bei Einwirken einer äußeren Kraft, ihre Festigkeit sprungartig zu erhöhen, sobald die äußere Kraft einen bestimmten Wert erreicht hat, und bei Nachlassen dieser äußeren Kraft wieder in den ursprünglichen Zustand zurückzukehren, die dabei über Dimensionen von weniger als 100$\mu$m verfügen kann, bereitzustellen.

Eine weitere Aufgabe der Erfindung ist es ein Verfahren zur Herstellung einer Schicht, die in der Lage ist, bei Einwirken einer äußeren Kraft, ihre Festigkeit sprungartig zu erhöhen, sobald die äußere Kraft einen bestimmten Wert erreicht hat, und bei Nachlassen dieser äußeren Kraft wieder in den ursprünglichen Zustand zurückzukehren, bereitzustellen.

Eine weitere Aufgabe der Erfindung ist es ein Verfahren zur Herstellung einer Schicht, die in der Lage ist, bei Einwirken einer äußeren Kraft, ihre Festigkeit sprungartig zu erhöhen, sobald die äußere Kraft einen bestimmten Wert erreicht hat, und bei Nachlassen dieser äußeren Kraft wieder in den ursprünglichen Zustand zurückzukehren (relaxieren), das eine Anpassung der Festigkeit an die wirkenden Kräfte, die erforderliche Festigkeit und das Relaxationsverhalten ermöglicht, bereitzustellen.

Die Aufgabe der Erfindung wird zum einen **gelöst** durch eine Schicht aufweisend vereinzelte in ihrer Länge ihren Durchmesser übertreffende säulenartige Elemente aus elastischem Material, die in einem Zentrum zu Zentrum Abstand voneinander auf einem Trägermaterial befestigt sind, **dadurch gekennzeichnet dass** die Elemente zueinander oder voneinander weg bewegbar sind, die Elemente sich beim zueinander bewegen berühren können, die Außenflächen der Elemente mit einer Oberflächenmodifikation versehen sind, die Oberflächenmodifikation Substanzen enthält, die zur Ausbildung reversibler, nicht kovalenter Bindungen befähigt sind.

Das Material weist in ihrer Länge ihren Durchmesser übertreffende Elemente (Säulen) aus einem Elastomer auf. Sie sind so beschaffen, dass eine äußere Krafteinwirkung auf die Elemente dafür sorgt, dass sich einzelne Teile der Elemente einander berühren.

Die Außenflächen der Elemente weisen eine Oberflächenmodifikation auf, die dafür sorgt, dass an den Stellen, an denen die Berührung stattfindet, eine reversible Verbindung der Teile entsteht. Die Stärke der Verbindung ist dabei

so gewählt, dass sie sich löst, sobald die zuvor auf das Material angewandte Kraft weggenommen wird und das Material in den Ausgangszustand zurückkehren kann (Reversibilität). Die Oberflächenmodifikation wird dabei nicht verändert.

Die Wahl des Trägermateriales bestimmt die initialen mechanischen Eigenschaften des Materials. Die Form das Trägermateriales und die Art der Oberflächenbehandlung bestimmen die mechanischen Eigenschaften des Materials im Fall der Verformung.

In einer Ausführungsform ist es bevorzugt ausschließlich biokompatible Materialien zu verwenden und somit ein biokompatibles Produkt zu erhalten.

Das Prinzip der erfindungsgemäßen Schicht ist schematisch in Abbildung 1 gezeigt. In Abbildung 1A sind die säulenartigen Elemente (1) mit einer Oberflächenmodifikation (2), die auf dem Trägermaterial (3) befestigt sind, ohne äußere Krafteinwirkung zu sehen. Die Struktur wird durch eine Deckplatte (4) abgedeckt. Durch äußere Krafteinwirkung (5) werden die Elemente geschert, die Elemente berühren sich (Abbildung 1B) aufgrund der Oberflächenmodifikation (2) kommt es zur Ausbildung reversibler nicht kovalenter Bindungen und damit zu einer Vernetzung und Versteifung des Materials. Beim Nachlassen oder Wegfall der äußeren Krafteinwirkung (5) kehrt das System aufgrund der rückstellenden Kraft durch die elastischen Eigenschaften der Elemente wieder in seinen ursprünglichen Zustand zurück (Abbildung 1C).

Es kann sich bei der Oberflächenmodifikation um eine Biofunktionalisierung, Chemische Funktionalisierung, Veränderung der Oberflächentopographie, um eine mechanische Vernetzung zu gewährleisten, handeln.

Die Oberflächenmodifikation (2) enthält Substanzen, die zur Ausbildung reversibler, nicht kovalenter Bindungen befähigt sind. Solche Substanzen sind dem Fachmann allgemein z.B. aus "Self-Healing Polymers: From Principles to Applications, First Edition. Edited by Wolfgang H. Binder © 2013 Wiley-VCH Verlag GmbH & Co. KGaA. Published 2013 by Wiley-VCH Verlag GmbH & Co. KGaA bekannt.

In einer besonderen Ausführungsform können die Substanzen, die zur Ausbildung reversibler, nicht kovalenter Bindungen befähigt sind, über eine Bindung zwischen Protein A und Antikörpern, Concanavalin A und $\alpha$-D-Glukose oder Streptavidin und Biotin verfügen. Solche Systeme sind dem Fachmann beispielsweise aus Kim et al. Biomicrofluidics 7, 041501 (2013) doi: 10.1063/1.4816934 und Pence et al. Biomater. Sci., 2014, 2, 1296-1304 (DOI: 10.1039/c4bm00193a) bekannt.

Des Weiteren kommen chemische Bindungskonzepte wie Supramolekulare Strukturen mit Wasserstoffbrückenbindungen, $\pi$-$\pi$-stacks, Metall-Ligand Systeme, oder Ionomere in Frage.

Die Elemente bestehen aus elastischen Materialien. Die Eigenschaft Elastizität lässt sich mit dem Elastizitätsmodul angeben. Elastizitätsmodule von Materialien findet der Fachmann beispielsweise in Materials Data Book 2003 Edition von Cambridge University Engineering Department.

Die spätere Anwendung der erfindungsgemäßen Schicht gibt die erforderlichen Parameter, mit Hilfe des erfindungsgemäßen Verfahrens erfolgt eine Auswahl der Materialien, Formen und Dimensionen der Elemente, sowie der Oberflächenmodifikation.

Zur Herstellung der erfindungsgemäßen Schicht wird ein Verfahren verwendet, dass zunächst die für die anschließende materielle Herstellung notwendigen Parameter festlegt, umfassend die folgenden Schritte:

i) Festlegung der Länge der Elemente (L) anhand der durch die spätere Anwendung vorgegebenen gesamten Dimensionierung und der erreichbaren Filmdicke;

ii) Durch die spätere Anwendung wird die grundlegenden Steifigkeit der Schicht vorgegebenen anhand dieser Information wird das Material (über den E-Modul), die Form und der Durchmesser der Elemente (über den Flächenträgheitsmodul I) festgelegt;

iii) Festlegung der gewünschten Auslenkung ($\Delta$) bis die Versteifung eintritt anhand der notwendigen prozentualen Versteifung der Schicht;

iv) Festlegung der Oberflächenmodifikation hinsichtlich der Substanzen und Konzentration der Substanzen unter der Maßgabe eine Haftkraft zu gewährleisten, die unterhalb der durch die Schritte i bis iii gegebenen Rückstellenden Kraft $R_A$ liegt;

v) Materielle Herstellung der Schicht.

Zur Materiellen Herstellung der Schicht können im Wesentlichen zwei Methoden verwendet werden.

Entweder die Säulenabstände sind direkt so festgelegt, dass der finale Säulenabstand mit einem Guss erreicht werden kann, oder es wird ein weiterer Säulenabstand gewählt, sodass der finale Säulenabstand durch das ineinander Stellen von zwei Proben erreicht wird.

Methode A - Fertige Schicht mit einem Guss:

Diese Methode ist für Oberflächenmodifikationen geeignet bei denen Substanzen eingesetzt werden sollen, die mit Ihresgleichen binden (z.B. Cadherin-Cadherin). Es darauf zu achten, dass beim Zusammengeben der Materialien die aktiven Enden der Substanzen, welche zur Oberflächenmodifikation eingesetzt werden, geblockt werden, damit die Moleküle an die Oberfläche binden und nicht nur mit sich selber reagieren. Die Abstände der säulenartigen Elemente sind nach Anwendung von Methode A immer gleich und es entfällt der Arbeitsschritt des Ineinanderstellens zweier Teilschichten.

Schritt v Methode A: Materielle Herstellung der Schicht umfassend die folgenden Schritte

- Herstellung der Gussform unter Berücksichtigung der in Schritt i bis iii festgelegten Parameter für die Länge, die Anordnung, die Form und den Durchmesser der Elemente nach dem Fachmann allgemein bekannten Methoden
- Aufbringen einer Antihaftbeschichtung auf die Gussform
- Abgießen der Gussform mit dem in Schritt ii festgelegten Material und E-Modul
- Aufbringen der Oberflächenmodifikation bei denen entsprechend Schritt iv Substanzen eingesetzt werden sollen, die mit Ihresgleichen binden (z.B. Cadherin-Cadherin).

Es ist darauf zu achten, dass beim Zusammengeben der Materialien die aktiven Enden der Substanzen, welche zur Oberflächenmodifikation eingesetzt werden, geblockt werden.

- Aufbringen eines Klebers
- Aufkleben der Deckplatte (4)
- Aushärten des Klebers

Methode B - Ineinanderstellen zweier Teilschichten:

Bei dieser Methode können Oberflächenmodifikationen gewählt werden, bei denen zwei verschiedene Substanzen, die miteinander eine reversible, nicht kovalente Bindung ausbilden (z.B. Biotin-Streptavidin) verwendet werden. Jeweils eine Teilschicht wird mit einer der beiden Substanzen funktionalisiert. Im Anschluss werden beide Teilschichten ineinander gestellt.

Schritt v Methode B: Materielle Herstellung der Schicht umfassend die folgenden Schritte

- Herstellung von ein bis zwei Gussformen unter Berücksichtigung der in Schritt i bis iii festgelegten Parameter für die Länge, die Anordnung, die Form und den Durchmesser der Elemente nach dem Fachmann allgemein bekannten Methoden
- Aufbringen einer Antihaftbeschichtung auf die Gussformen
- Abgießen einer Gussform mit dem in Schritt ii festgelegten Material und E-Modul
- Abgießen einer weiteren Gussform mit dem in Schritt ii festgelegten Material und E-Modul
- Aufbringen der Oberflächenmodifikation bei denen entsprechend Schritt iv zwei verschiedene Substanzen eingesetzt werden, die miteinander eine reversible, nicht kovalente Bindung ausbilden
- Aufbringen des Klebers
- Ineinanderstellen der beiden Teilschichten
- Aushärten des Klebers

Die Herstellung der Gussform erfolgt nach dem Fachmann allgemein bekannten Methoden. Neben dem Verfahren des Reaktiven Ionenätzens kommt die Lithographie insbesondere die Fotolithographie zum Einsatz.

Antihaftbeschichtungen sind dem Fachmann allgemein bekannt, bevorzugt wird eine Silansierung verwendet.

Das Abgießen der Gussform erfolgt nach allgemein bekannten Methoden. Bevorzugt werden Monomere oder Präpolymere gemeinsam mit einem geeigneten "Vernetzer" in die Form eingeführt und so auf chemischem Wege über Polyreaktionen ausgehärtet. Bei Verwendung dieser Methode kann durch die Menge an "Vernetzer" der E-Modul des Materials auf den zuvor festgelegten Wert eingestellt werden. Eine Aushärtung kann auch auf anderen Wegen dem Fachmann allgemein bekannten Wegen z.B. über eine Lichtinduzierte Polymerisation erfolgen.

Beim Aufkleben der Deckplatte (4) hat es sich als besonders vorteilhaft erwiesen als "Kleber" unvernetztes Material der gleichen Art wie zum Abgießen der Gussform zu verwenden. Dabei wird folgendermaßen vorgegangen:

- Eintauchen der Säulenspitzen in noch unvernetztes Monomer oder Präpolymer - gegebenenfalls unter Zugabe des Vernetzers als "Kleber", hierbei sollte darauf geachtet werden, dass nur die Spitzen in den Kleber eintauchen können. Dies lässt sich zum Beispiel dadurch erreichen, dass das Monomer oder Präpolymer durch Spincoating auf einer Glasplatte so dünn gemacht wird, dass nur ein Teil der Säulen eintauchen kann (Schichtdicke kann bis 1 µm oder dicker sein);
- Aufkleben einer Deckschicht z.B. aus Monomer oder Präpolymer oder Glas.

Das Aushärten des Klebers kann bei Raumtemperatur oder - wenn die gewählte Oberflächenmodifikation dieses zulässt - durch Erwärmen oder Bestrahlung mit einer geeigneten Lichtquelle erfolgen oder beschleunigt werden.

Im Folgenden soll das Erfindungsgemäße Verfahren genauer erläutert werden, sowie einige Beispiele aufgezeigt werden. Eine graphische Darstellung der Zusammenhänge findet sich in Abbildung 2.

Die Rückstellkraft RA der Säulen berechnet sich aus $R_A = \frac{EI\Delta}{L^3}$ mit

- E: E-Modul des Kunststoffes
- I: Flächenträgheitsmodul 2ten Grades
- Δ: Verschiebung der Säulen
- L: Länge der Säulen

Variationen in der Form der Grundfläche der Säulen stecken im Flächenträgheitsmodul I:

$$\text{z.B.: } I_{rund} = \frac{\pi \cdot d^4}{64}\ ;\ I_{quadrat} = \frac{h^4}{12}\ ;\ I_{rechteck} = \frac{b \cdot h^3}{12}\ (mit\ h\text{: }k\ddot{u}rzere\ Seite)$$

Der Rückstellkraft $R_A$ wirkt die Haftkraft der Bindungen entgegen.

Die Haftkraft wird berechnet, indem die Kontaktfläche der säulenartigen Elemente miteinander bestimmt wird. Da die säulenartigen Elemente sowohl oben, als auch unten einen definierten Abstand zueinander haben, muss eine Annahme getroffen werden, welcher Teil der Länge eines säulenartigen Elements Kontakt zum nächsten hat. Anschließend wird mit Hilfe der Fläche, die jeder Bindungspartner einnimmt bestimmt, wie viele Bindungspartner miteinander in Kontakt stehen. Die Kraft einer einzelnen Bindung ist bekannt. So lässt sich die maximale Bindungskraft pro Fläche bestimmen. Es ist durch Modifikationen in der Funktionalisierung der Oberfläche möglich, weniger Bindungspartner an die Oberfläche zu binden und somit auch eine geringere Haftkraft zwischen den säulenartigen Elementen zu erreichen.

Wählbare Parameter sind somit mit Einfluss auf die Rückstellkraft:

- Länge der Pillars (Einfluss auf L)
- Grundfläche der Pillars (Einfluss auf I)
- Form der Pillars (Einfluss auf I)
- Material der Pillars (Einfluss auf E)

Mit Einfluss auf die Haftkraft:

- Form der Pillars (Einfluss auf Kontaktfläche)
- Art der Bindung (Stärke der Bindung pro Flächeneinheit)
- Anzahl der Bindungen pro Fläche (Maximum ist definiert. Verminderung möglich)

Mögliche Kombinationen aus Materialien und Formen wären z.B.

1: Beispiel für ein weiches Polymer:

Material: Chloropren-Kautschuk (CR, Neopren): E=700kPa

Form der Säulen: rund

Länge der Säulen: 15 μm

Durchmesser der Säulen: 5 μm

Verschiebung der Säulen: 8 μm

Rückstellkraft: 6,1E-7 N

Bindung: Streptavidin - Biotin (ca. 150 pN)

Haftkraft: ca. 4,2E-7 N

Hier wurde angenommen, dass die Kontaktfläche zwischen den Pillars 50% der Länge und 2nm in der Breite beträgt.

2: Beispiel für ein extrem weiches Polymer:

Material: Polyacrylamide Hydrogel: E=10kPa

Form der Säulen: rund

Länge der Säulen: 15 μm

Durchmesser der Säulen: 5 μm

Verschiebung der Säulen: 8 μm

Rückstellkraft: 8,7E-9 N

Bindung: Concanavalin A- Mannose Kohlehydrat (ca. 47 pN)

Haftkraft: ca. 8,4E-9 N (bei ca. 6,25% der maximalen Oberflächenbedeckung mit Con A)

Hier wurde angenommen, dass die Kontaktfläche zwischen den Pillars 50% der Länge und 2nm in der Breite beträgt.

3: Beispiel für sehr hartes Polymer:

Material: Silikon Elastomer: E=20000kPa

Form der Säulen: Quadratisch

Länge der Säulen: 15 $\mu$m

Seitenlänge der Säulen: 5 $\mu$m

Verschiebung der Säulen: 8 $\mu$m

Rückstellkraft: 2,9E-5 N

Bindung: Concanavalin A- Mannose Kohlehydrat (ca. 47 pN)

Haftkraft: ca. 2,3E-5 N (bei ca. 10% der maximalen Oberflächenbedeckung mit Con A)

Hier wurde angenommen, dass die Kontaktfläche zwischen den Säulen 50% der Länge und die gesamte Breite der Säulen beträgt.

4: Beispiel für Cadherin Catch Bonds:

Material: Silikon Elastomer: E=20000kPa

Form der Säulen: Quadratisch

Länge der Säulen: 15 $\mu$m

Seitenlänge der Säulen: 5 $\mu$m

Verschiebung der Säulen: 8 $\mu$m

Rückstellkraft: 2,9E-5 N

Bindung: Cadherin Catch Bonds (ca. 27-32 pN)

Haftkraft: ca. 2,3E-5 N - 3,7E-5 (bei ca. 80% der maximalen Oberflächenbedeckung mit Cadherin)

Hier wurde angenommen, dass die Kontaktfläche zwischen den Säulen 50% der Länge und die gesamte Breite der Säulen beträgt.

5: Beispiel für sehr große Säulen:

Material: Silikon Elastomer: E=20000kPa

Form der Säulen: Quadratisch

Länge der Säulen: 400 $\mu$m

Seitenlänge der Säulen: 100 $\mu$m

Verschiebung der Säulen: 160 $\mu$m

Rückstellkraft: 5E-3 N

Bindung: Concanavalin A- Mannose Kohlehydrat (ca. 47 pN)

Haftkraft: 4,9E-3N (bei ca. 4% der maximalen Oberflächenbedeckung mit Con A)

Hier wurde angenommen, dass die Kontaktfläche zwischen den Säulen 50% der Länge und die gesamte Breite der Säulen beträgt.

Da hier jeweils die maximalen Kräfte der Bindungen gegeben sind, besteht die Möglichkeit mit einer höheren Konzentration an Oberflächenmodifikation, oder weicheren Polymeren zu arbeiten und somit Unterschiede auszugleichen.

Bevorzugte E-Module der Elastomere liegen zwischen 10kPa und 20000kPa variieren.

Die Stärke der Bindungen variiert zwischen 5pN für eine Wasserstoffbrückenbindung bis hin zu 150pN für die Streptavidin-Biotin Bindung.

Cadherin Catch Bonds variieren zwischen 27 und 32 pN.

Der bevorzugte Durchmesser der Säulen, liegt zwischen 4 und 100 μm. Die bevorzugte Länge zwischen 10 und 400 μm und der bevorzugte Zentrum-Zentrum Abstand bei 8,8 bis 220 μm.

Es ist bevorzugt, dass der Abstand der Säulenseiten zueinander ca. 10% der Säulendicke beträgt.

Das Länge zu Dicke Verhältnis der Säulen sollte bevorzugt maximal 4:1 betragen.

Weitere Informationen zur tiefergehenden Erläuterung:

Es lässt sich eine Formel (Kurvenfunktion) für die Biegelinie der Säulen finden.

$$w(x) = S_Q \cdot \left\{ 3 \cdot \left[\frac{x}{L_0}\right]^2 - 2 \cdot \left[\frac{x}{L_0}\right]^3 \right\}$$

*mit $S_q$: Verschiebung der Säulen und $L_0$ : Länge der Säulen*

Mit Hilfe dieser kann bestimmt werden, bei welcher Verschiebung/Biegung ein Kontakt zwischen den Säule auftritt. Es ist zu beachten, dass die Kurve für die neutrale Faser in der Mitte der Säule beschrieben wird. Die Kurve der Außenkanten wird mit Hilfe entsprechender Parallelkurven zu der Kurve in der Mitte bestimmt. Wird dies durchgerechnet, zeigt sich, dass für das Beispielsystem mit Säulen von 5μm Durchmesser, 15μm Länge und einem Zentrum-Zentrum Abstand von 5,5μm bei einer Biegung von 8μm eine Kontaktfläche von ca. 50% der Säulenlänge vorliegt.

Haften 50% der Säulenlänge von quadratischen Säulen aneinander, so kann man das daraus resultierende System folgendermaßen beschreiben:

Der nicht haftende Teil jeder Säule (ein Teil oberhalb des haftenden Teiles und ein Teil unterhalb) ist eine Säule mit einem Viertel der Ausgangslänge.

Es sind in diesem System also zwei Säulen mit einem Viertel der Ausgangslänge entstanden. Beide Säulen müssen für eine Verschiebung des Systems um die Länge x um die Länge x/2 verschoben werden. Da die Rückstellkraft mit $1/L^3$ von der Säulenlänge abhängt, wird die Kraft, die nach dem Haften der Säulen benötigt wird, um das System zu verbiegen 64 Mal so hoch sein, wie zuvor.

Im Folgenden soll exemplarisch die Erzeugung einer erfindungsgemäßen Schicht nach Festlegung der Parameter entsprechend des Verfahrens beschrieben werden.

Für dieses Beispiel wird auf einer Fläche von 3x3mm eine hexagonale Anordnung von runden Säulen gewählt, die einen Durchmesser von 4,5 μm und Zentrum zu Zentrum Abstand von 10 μm aufweisen.

Material: Polydimethylsiloxan (PDMS) E-Modul E=1720 kPa (aus Palchesko doi:10.1371/journal.pone.0051499)

Form der Säulen: rund

Länge der Säulen: 15 μm

Durchmesser der Säulen: 4,5 μm

Verschiebung der Säulen: 6,5 μm

Rückstellkraft: 3,26E-7

Bindung: Biotin Streptavidin (ca. 150 pN)

Haftkraft: 3,20E-7 (bei 75% der maximalen Oberflächenbedeckung mit Streptavidin)

Hier wurde angenommen, dass die Kontaktfläche zwischen den Pillars 50% der Länge und 2nm in der Breite beträgt.

Zunächst erfolgt die Herstellung der Gussform. Auf einen 4" Silizium Wafer wird per Spincoating der Fotolack SU-8 10 von Microchem aufgetragen. Die Dicke des Lackes bestimmt die Länge der späteren Säulen. Verschiedene Lacke von Microchem können unterschiedliche Schichtdicken Darstellen. Für die hier gewählte Schichtdicke von 15μm eignet sich der SU-8 10 sehr gut.

In einem Lithographieprozess werden Löcher in diesem Fotolack erzeugt, die dem Negativ der späteren Säulen entsprechen. Die Strukturen auf der Fotomaske bestimmen somit die Anordnung und die Form der Löcher im Fotolack, die später den Säulen entsprechen.

Um späteres Anhaften des PDMS an der Form zu verhindern, wird eine Antihaftschicht aufgetragen, in diesem Fall wurde aus der Gasphase eine Monolage Trichloro (1H, 1H, 2H, 2H-perfluorooctyl)silane abgeschieden.

Nun erfolgt das Abgießen der Gussform mit PDMS. PDMS (Sylgard 184 von Dow) welches als Zwei-Komponenten Kit, bestehend aus Grundpolymer und Vernetzer, angeboten wird, wird im Verhältnis (10:1 w/w Polymer:Vernetzer) angemischt. Dies wird vom Hersteller so empfohlen. Andere Verhältnisse führen zu anderen Werten für den E-Modul. Je mehr Vernetzer verwendet wird, desto härter wird das Elastomer. Durch weniger Vernetzer wird das Polymer weicher. Das heißt das E-Modul wird geringer.

Das angemischte Polymer wird auf eine Menzel Deckglas gegeben und die Gussform draufgelegt. Anschließend wird das Polymer für eine Stunde bei 100°C ausgehärtet.

Der Vorgang wird zur Herstellung einer identischen Teilschicht wiederholt.

Abb. 3 zeigt eine Elektronenmikroskopische Aufnahme der erzeugten Säulen mit einem Durchmesser von 4,5 μm und einer Zentrum zu Zentrum Distanz von 10 μm.

Eine Teilstruktur wird entsprechend des folgenden Reaktionschemas mit Biotin beschichtet.

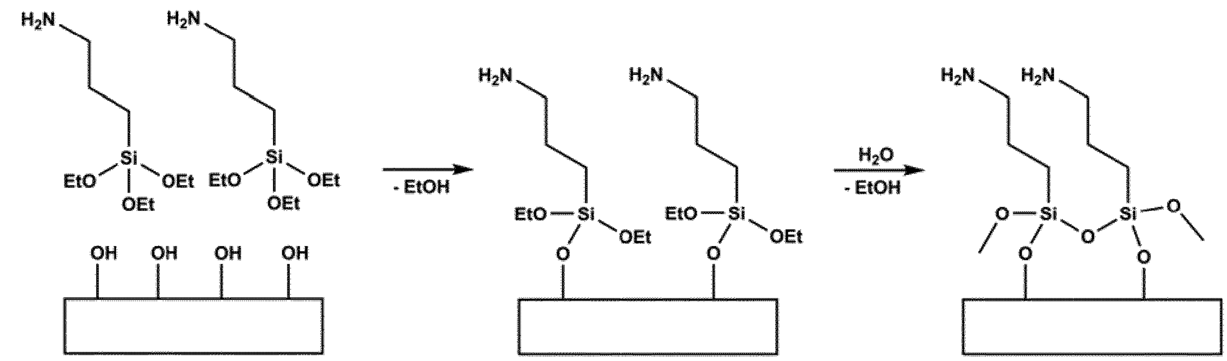


Silanisierung mit (3-Aminopropyl)triethoxysilane (APTES).

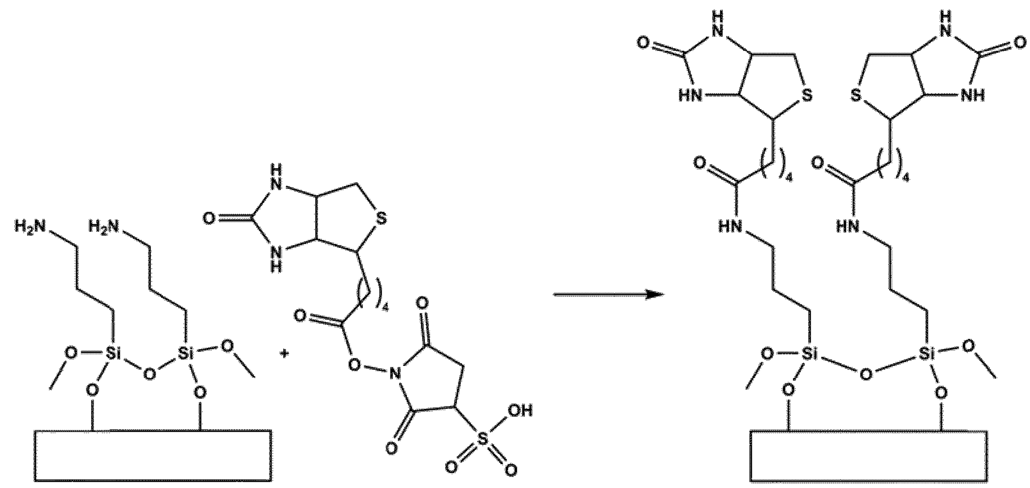

Anbindung des Biotins

Das Polymer wird zunächst im Sauerstoffplasma aktiviert und mit (3-Aminopropyl)triethoxysilane (APTES)

silanisiert.

Biotin 3-sulfo-N-hydroxysuccinimide ester sodium salt wird anschließend via Bildung eines Amids kovalent an die Aminogruppen des APTES angebunden.

Die zweite Teilstruktur wird zwecks Herstellung der passenden Teilschicht mit wird entsprechend Kim et al. Biomicrofluidics 7, 041501 (2013) mit Streptavidin beschichtet. Hier wird nach einer Plasmabehandlung (3-mercaptopropyl)trimethoxysilan für eine Silanisierung gewählt. Biotin 3-sulfo-N-hydroxysuccinimide ester sodium salt wird anschließend via Bildung eines Amids kovalent an die Aminogruppen des APTES angebunden. Nach Mudraboyina et al. Sensors 2011, 11, 11295-11304; doi:10.3390/s111211295, ebenfalls in Kim et al. Biomicrofluidics 7, 041501 (2013), wird Glutaraldehyd benutzt, um zwischen den Aminogruppen im Protein (Streptavidin) und auf der Oberfläche eine kovalente Bindung aufzubauen. Hierbei wird die Funktionalität des Streptavidins nicht beeinflusst.

Beide Strukturen werden nach vorherigem Eintauchen der Säulenspitzen in dünn auf einen Wafer verteiltem nicht ausgehärteten PDMS ineinander gestellt und ausgehärtet. Der Ablauf ist in Abbildung 4 schematisch dargestellt.

Das Ziel dieses Schrittes besteht darin mit Biotin beschichtete Säulen (7) und mit Streptavidin beschichtete Säulen (8) so nah wie möglich aneinander zu bringen (9). Dies ist dadurch begründet, dass es nicht möglich ist nur jeweils eine Säule mit Biotin und eine mit Streptavidin zu coaten. Aus diesem Grund wird jeweils eine Oberflächenmodifikation pro Teilstruktur durchgeführt und dann zwei Teilschichten ineinander gestellt. Wenn man sich die hexagonale Struktur mit Säulenabständen von 10μm Zentrum zu Zentrum, dann ist der Abstand zwischen den Säulenwänden genau 5,5μm. Wenn man jetzt die Streptavidin Struktur genau in die Lücken zwischen den Biotin Säulen stellt, dann hat man Reihen mit jeweils 0,5μm Abständen zwischen jeweils einer Biotin-Säule und einer Stretavidin-Säule. Diese können sich dann bei Einwirken einer Kraft und Auslenkung berühren. Biotin und Streptavidin binden reversibel aneinander.

Als "Kleber" (6) wird unvernetztes PDMS verwendet. Dieses verbindet sich beim Aushärten perfekt mit dem PDMS der Säulen.

Nachfolgend werden Ausführungsbeispiele der Erfindung anhand der beiliegenden Zeichnungen in der **Figurenbeschreibung** detailliert beschrieben, wobei diese die Erfindung erläutern sollen und nicht beschränkend zu werten sind:
Es zeigen:

Fig. 1 Prinzip der erfindungsgemäßen Schicht;

Fig. 2 Graphische Darstellung der für das erfindungsgemäße Verfahren wichtigen Parameter;

Fig. 3 Elektronenmikroskopische Aufnahmeeiner Säulenstruktur aus PDMS. Die säulenförmigen Elemente weisen einen Durchmesser von 4,5 μm und eine Zentrum zu Zentrum Distanz von 10 μm auf.

Fig. 4 Zusammenführung zweier Strukturen mit einem Säulendurchmesser von 4,5 μm und einer Zentrum zu Zentrum Distanz von 10 μm zu einer erfindungsgemäßen Schicht mit 0,5μm Abstand zwischen den Säulen.

Bezugszeichenliste

| | | | |
|---|---|---|---|
| 1 | Säulenartigen Elemente | 2 | Oberflächenmodifikation |
| 3 | Trägermaterial | 4 | Deckplatte |
| 5 | Äußere Krafteinwirkung | 6 | Kleber |
| 7 | Biotin Beschichtung | 8 | Streptavidin Beschichtung |
| 9 | Biotin und Streptavidin beschichtete Säulen in geringem Abstand | | |

## Patentansprüche

1. Schicht aufweisend vereinzelte in ihrer Länge ihren Durchmesser übertreffende Elemente aus elastischem Material, die in einem Abstand voneinander auf einem Träger befestigt sind,
**dadurch gekennzeichnet, dass**
die Elemente zueinander oder voneinander weg bewegbar sind, die Elemente sich beim zueinander bewegen berühren können, die Außenflächen der Elemente mit einer Oberflächenmodifikation versehen sind, die Oberflächenmodifikation Substanzen enthält, die zur Ausbildung reversibler, nicht kovalenter Bindungen befähigt sind.

2. Schicht nach dem vorangehenden Anspruch,
**dadurch gekennzeichnet, dass**

die Substanzen ausgewählt sind aus der Gruppe Protein A und Antikörper, Concanavalin A und α-D-Glukose oder Streptavidin und Biotin.

3. Schicht nach einem der vorangehenden Ansprüche 1 bis 2,
**dadurch gekennzeichnet, dass**
die Substanzen ausgewählt sind aus Cadherin-Cadherin.

4. Verfahren zur Herstellung einer Schicht nach einem der vorangehenden Ansprüche, **umfassend die Schritte,**

i Auswahl der Länge der Elemente (L) anhand der durch die spätere Anwendung vorgegebenen gesamten Dimensionierung;
ii Auswahl des Materials (E-Modul), der Form und des Durchmessers der Elemente (Flächenträgheitsmodul I) anhand der durch die spätere Anwendung vorgegebenen grundlegenden Steifigkeit der Schicht;
iii Auswahl der gewünschten Auslenkung (Δ) bis die Versteifung eintritt und damit Auswahl der gewünschten prozentualen Versteifung der Schicht;
iv Auswahl der Oberflächenmodifikation hinsichtlich der Substanzen und Konzentration der Substanzen unter der Maßgabe eine Haftkraft zu gewährleisten, die unterhalb der durch die Schritte i bis iii gegebenen Rückstellenden Kraft $R_A$ liegt;
v Materielle Herstellung der Schicht.

5. Verfahren nach Anspruch 4,
**dadurch gekennzeichnet, dass**
die materielle Herstellung (v) die folgenden Schritte umfasst (Methode A)

- Herstellung der Gussform unter Berücksichtigung der in Schritt i bis iii festgelegten Parameter für die Länge, die Anordnung, die Form und den Durchmesser der Elemente nach dem Fachmann allgemein bekannten Methoden;
- Aufbringen einer Antihaftbeschichtung auf die Gussform;
- Abgießen der Gussform mit dem in Schritt ii festgelegten Material und E-Modul;
- Aufbringen der Oberflächenmodifikation, bei denen - entsprechend Schritt iv- Substanzen eingesetzt werden sollen, die mit Ihresgleichen binden (z.B. Cadherin-Cadherin), wobei darauf zu achten ist, dass beim Zusammengeben der Materialien die aktiven Enden der Substanzen, welche zur Oberflächenmodifikation eingesetzt werden, geblockt werden;
- Aufbringen eines Klebers;
- Aufkleben der Deckplatte (4);
- Aushärten des Klebers.

6. Verfahren nach Anspruch 4 oder 5,
**dadurch gekennzeichnet, dass**
die materielle Herstellung (v) die folgenden Schritte umfasst (Methode B)

- Herstellung von ein bis zwei Gussformen unter Berücksichtigung der in Schritt i bis iii festgelegten Parameter für die Länge, die Anordnung, die Form und den Durchmesser der Elemente nach dem Fachmann allgemein bekannten Methoden;
- Aufbringen einer Antihaftbeschichtung auf die Gussformen;
- Abgießen einer Gussform mit dem in Schritt ii festgelegten Material und E-Modul;
- Abgießen einer weiteren Gussform mit dem in Schritt ii festgelegten Material und E-Modul;
- Aufbringen der Oberflächenmodifikation bei denen - entsprechend Schritt iv - zwei verschiedene Substanzen eingesetzt werden, die miteinander eine reversible, nicht kovalente Bindung ausbilden;
- Aufbringen des Klebers;
- Ineinanderstellen der beiden Teilschichten;
- Aushärten des Klebers.

7. Verwendung einer Schicht nach einem der Ansprüche 1 bis 3 als schockabsorbierendes Mittel oder für flexible Anzeigeanordnungen für sich ändernde Informationen (Displays).

8. Schicht nach einem der Ansprüche 1 bis 3 zur Verwendung als Gefäßprothese mit nicht linearem elastischen

Verhalten.

## Claims

1. Layer having isolated elements whose length is greater than their diameter and which are made of a resilient material and fastened to a substrate at a spacing from one another,
**characterised in that**
the elements are movable towards or away from one another, the elements can touch one another when they move towards one another, the outer surfaces of the elements are provided with a surface modification, the surface modification contains substances that are capable of forming reversible, non-covalent bonds.

2. Layer according to the preceding claim,
**characterised in that**
the substances are selected from the group of protein A and antibodies, concanavalin A and $\alpha$-D-glucose or streptavidin and biotin.

3. Layer according to any of the preceding claims 1 to 2,
**characterised in that**
the substances are selected from cadherin-cadherin.

4. Method for producing a layer according to any of the preceding claims, comprising the steps of:

   i selecting the length of the elements (L) on the basis of the overall dimensions predetermined by the later use;
   ii selecting the material (elastic modulus), the shape and the diameter of the elements (area moment of inertia I) on the basis of the basic rigidity of the layer predetermined by the later use;
   iii selecting the desired deflection ($\Delta$) before the stiffening occurs and thus selecting the desired percentage stiffening of the layer;
   iv selecting the surface modification in terms of the substances and concentration of the substances, with the proviso that an adhesive force that is less than the restoring force $R_A$ provided by steps i to iii is ensured;
   v physical production of the layer.

5. Method according to claim 4,
**characterised in that**
the physical production (v) comprises the following steps (method A):

   - producing the casting mould, taking account of the parameters for the length, arrangement, shape and diameter of the elements as specified in steps i to iii, in accordance with the methods generally known to the person skilled in the art;
   - applying an anti-stick coating to the casting mould;
   - filling the casting mould with the material and elastic modulus specified in step ii;
   - applying the surface modification, in which, in accordance with step iv, substances that bind to their own kind (e.g. cadherin-cadherin) are to be used, whereby it should be ensured that the active ends of the substances used for the surface modification are blocked when the materials are mixed;
   - applying an adhesive;
   - gluing on the cover plate (4);
   - curing the adhesive.

6. Method according to either claim 4 or claim 5,
**characterised in that**
the physical production (v) comprises the following steps (method B):

   - producing one to two casting moulds, taking account of the parameters for the length, arrangement, shape and diameter of the elements as specified in steps i to iii, in accordance with the methods generally known to the person skilled in the art;
   - applying an anti-stick coating to the casting moulds;
   - filling one casting mould with the material and elastic modulus specified in step ii;
   - filling an additional casting mould with the material and elastic modulus specified in step ii;

- applying the surface modification, in which, in accordance with step iv, two different substances that form a reversible, non-covalent bond with one another are used;
- applying the adhesive;
- fitting the two part-layers together;
- curing the adhesive.

**7.** Use of a layer according to any of claims 1 to 3 as a shock-absorbing means or for flexible display arrangements for changing information (displays).

**8.** Layer according to any of claims 1 to 3 for use as a vascular prosthesis having nonlinear elastic behaviour.

## Revendications

**1.** Couche composée d'éléments individuels en matériau élastique dont la longueur dépasse le diamètre, et qui sont fixés à distance les uns des autres sur un support, **caractérisée en ce que** les éléments peuvent être rapprochés ou éloignés les uns des autres, les éléments peuvent se toucher lorsqu'ils se rapprochent, les surfaces extérieures des éléments sont pourvues d'une modification de surface, la modification de surface contient des substances qui sont capables de former des liaisons réversibles et non covalentes.

**2.** Couche selon la revendication précédente, **caractérisée en ce que** les substances sont choisies dans le groupe constitué par la protéine A et des anticorps, la concanavaline A et l'a-D-glucose ou la streptavidine et la biotine.

**3.** Couche selon l'une des revendications 1 à 2 précédentes, **caractérisée en ce que** les substances sont choisies parmi la cadhérine-cadhérine.

**4.** Procédé de fabrication d'une couche selon l'une des revendications précédentes, **comprenant les étapes** consistant en :

i la sélection de la longueur des éléments (L) en fonction du dimensionnement global prédéterminé par l'application ultérieure ;
ii la sélection du matériau (module d'élasticité), de la forme et du diamètre des éléments (moment quadratique I) en fonction de la raideur de base de la couche prédéterminée par l'application ultérieure ;
iii la sélection de la déformation souhaitée ($\Delta$) jusqu'à ce que le raidissement se produise et ainsi la sélection du pourcentage de raidissement souhaité de la couche ;
iv la sélection de la modification de surface en ce qui concerne les substances et la concentration des substances afin de garantir une force d'adhérence qui soit inférieure à la force de rappel $R_A$ donnée par les étapes i à iii ;
v la fabrication effective de la couche.

**5.** Procédé selon la revendication 4, **caractérisé en ce que** la fabrication effective (v) comprend les étapes suivantes (méthode A) consistant en

- la production du moule, en tenant compte des paramètres pour la longueur, la disposition, la forme et le diamètre des éléments tels que définis dans les étapes i à iii, selon des méthodes généralement connues de l'homme du métier ;
- l'application d'un revêtement antiadhésif sur le moule ;
- le remplissage du moule avec le matériau et le module d'élasticité définis à l'étape iv ;
- l'application de la modification de surface dans laquelle, selon l'étape iv, il faut utiliser des substances qui se lient avec leurs homologues (par exemple cadhérine-cadhérine), en s'assurant, lors de l'ajout des matériaux, que les extrémités actives des substances utilisées pour la modification de la surface soient bloquées ;
- l'application d'un adhésif ;
- le collage de la plaque de recouvrement (4) ;
- le durcissement de l'adhésif.

**6.** Procédé selon la revendication 4 ou 5, **caractérisé en ce que** la fabrication effective (v) comprend les étapes suivantes (procédé B) consistant en :

- la fabrication d'un ou de deux moules, en tenant compte des paramètres de longueur, de disposition, de forme

et de diamètre des éléments tels que définis dans les étapes ***i*** à iii, selon des méthodes généralement connues de l'homme du métier ;
- l'application d'un revêtement antiadhésif sur les moules ;
- le remplissage d'un moule avec le matériau et le module d'élasticité définis dans l'étape ii ;
- le remplissage d'un autre moule avec le matériau et le module d'élasticité définis à l'étape ii ;
- l'application de la modification de surface dans laquelle - selon l'étape ***iv*** - deux substances différentes sont utilisées qui forment une liaison réversible et non covalente l'une avec l'autre ;
- l'application de l'adhésif ;
- le placement l'une dans l'autre des deux couches partielles ;
- le durcissement de l'adhésif.

**7.** Utilisation d'une couche selon l'une des revendications 1 à 3 comme moyens d'absorption des chocs ou pour des dispositions d'affichage flexibles pour informations pouvant être modifiées (écrans).

**8.** Couche selon l'une des revendications 1 à 3 pour une utilisation en tant que prothèse vasculaire à comportement élastique non linéaire.

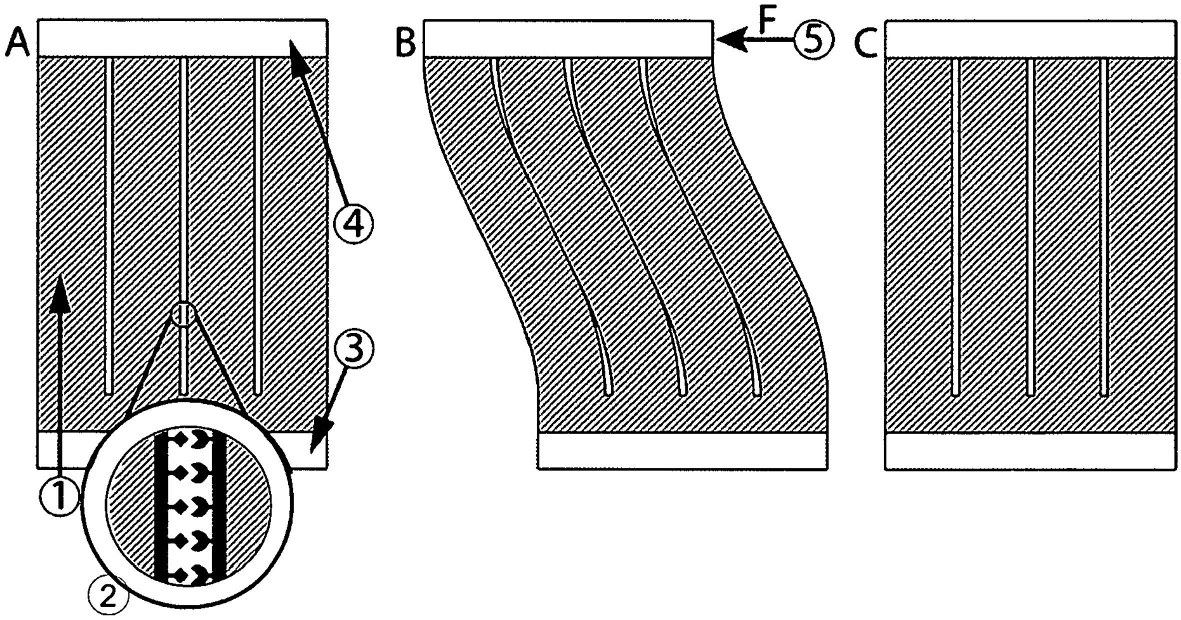


Abb. 1

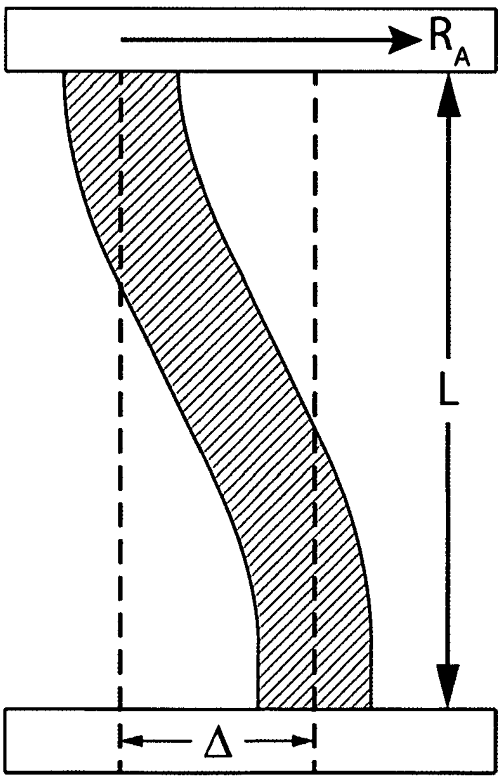


Abb. 2

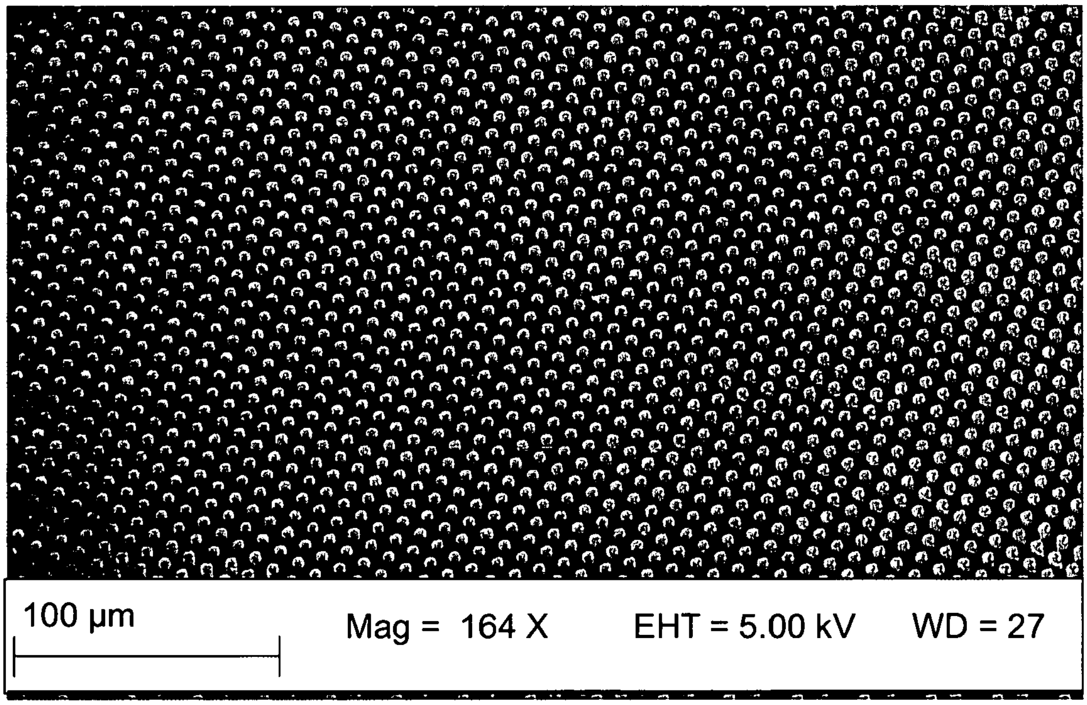


Abb. 3

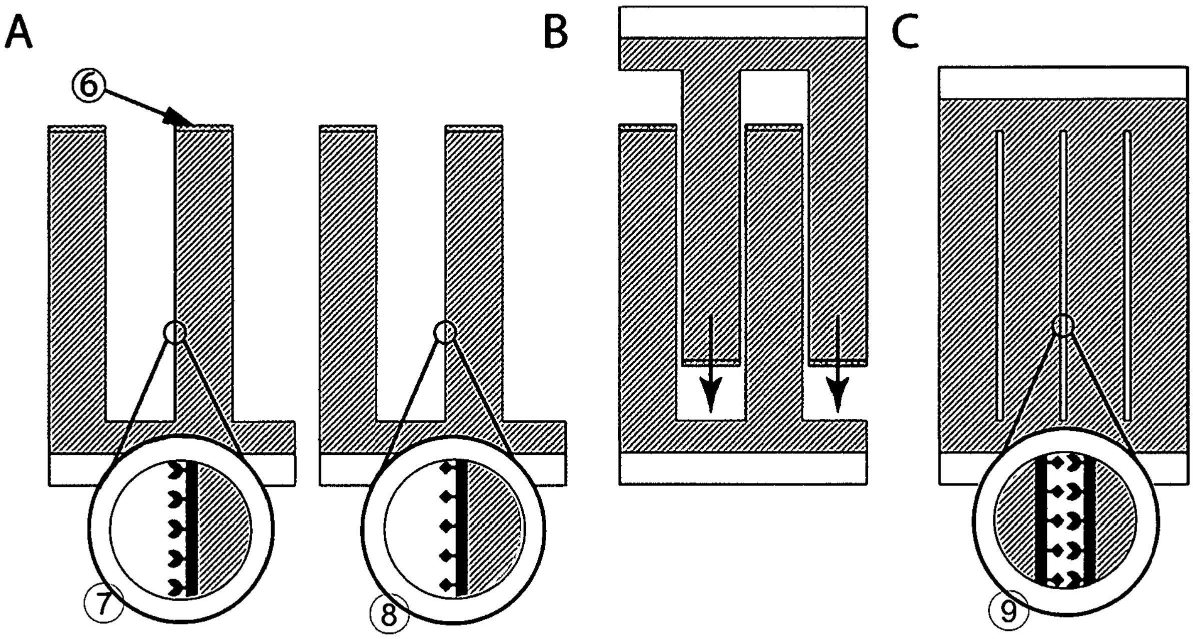


Abb. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20030055496 A1
- US 20100205722 A
- DE 60222805 T2
- US 8072437 B2


### In der Beschreibung aufgeführte Nicht-Patentliteratur


- **QI WEN ; PAUL A. JANMEY.** *Effects of non-linearity on cell-ECM interactions, Experimental Cell Research,* 01. Oktober 2013, vol. 319 (16), ISSN 0014-4827, 2481-2489, http://dx.doi.org/10.1016/j.yexcr.2013.05.017
- Self-Healing Polymers: From Principles to Applications. Wiley-VCH Verlag GmbH & Co. KGaA, 2013
- **KIM et al.** *Biomicrofluidics,* 2013, vol. 7, 041501
- **PENCE et al.** *Biomater. Sci.,* 2014, vol. 2, 1296-1304
- Self-Healing Polymers: From Principles to Applications. Wiley-VCH Verlag GmbH & Co. KGaA, 2013
- Materials Data Book. Cambridge University Engineering Department, 2003
- **NACH MUDRABOYINA et al.** *Sensors,* 2011, vol. 11, 11295-11304